# EUROPÄISCHE PATENTSCHRIFT

(11) **EP 0 589 082 B1**
(45) Veröffentlichungstag und Bekanntmachung des Hinweises auf die Patenterteilung: **14.04.1999**
(21) Anmeldenummer: 92116361.4
(22) Anmeldetag: 24.09.1992
(51) Int. Cl.: G10K 11/00, A61B 8/12

(54) **Intrakavitäre Ultraschallsonde**
Intracavitary ultrasonic probe
Sonde intracavitaire à ultrasons

(43) Veröffentlichungstag der Anmeldung: 30.03.1994
(73) Patentinhaber: SIEMENS AKTIENGESELLSCHAFT, 80333 München (DE)
(72) Erfinder: Pohan, Claus, Dipl.-Ing. (FH), W-8523 Baiersdorf (DE); Schmitt, Karl-Jürgen, Dr. rer. nat., W-8600 Bamberg (DE)

(56) Entgegenhaltungen:
- EP-A- 0 359 546
- DE-A- 2 504 225
- US-A- 4 543 960
- US-A- 4 841 979
- US-A- 5 090 414

## Beschreibung

Die Erfindung betrifft eine intrakavitäre Ultraschallsonde zur Erzeugung von Schnittbildern eines Untersuchungsgebietes mit einem Einführabschnitt, der distal in einem Endteil endet, welches Endteil ein konvexes Wandlerarray umfaßt.

Eine intrakavitäre Ultraschallsonde der eingangs genannten Art für Prostatauntersuchungen ist in der EP-A-0 446 645 beschrieben. Die Prostatasonde ermöglicht es, die Prostata vom Rektum aus mit Ultraschallstrahlen abzutasten und aus den Echosignalen Schnittbilder der Prostata zu erzeugen. Sie besteht aus einem geraden Einführabschnitt, an dessen distalem Ende ein gebogenes Endteil angeordnet ist. Das gebogene Endteil hat eine äußere Oberfläche, die konvex geformt ist und in der ein konvexes Wandlerarray angeordnet ist. Die Wandlerelemente des konvexen Wandlerarrays werden fortschaltend gruppenweise aktiviert, um eine Sektorabtastung durchzuführen. Der besondere Vorteil eines konvexen Wandlerarrays besteht darin, daß die davon erzeugten Ultraschall-Schnittbilder schon im Nahbereich des Wandlerarrays eine gute bildliche Auflösung von Details zeigen. Gleichzeitig bieten konvexe Wandlerarrays ein großes Abtastfeld, so daß Schnittbilder der gesamten Prostata erzeugt werden können.

Aus diagnostischen und/oder therapeutischen Gründen wäre es wünschenswert, wenn das Untersuchungsgebiet nicht nur in einer Schnittebene, sondern in mehreren verschiedenen Schnittebenen darstellbar wäre, die einen gemeinsamen Ursprung haben. Besonders hilfreich wäre eine Darstellung des Untersuchungsgebietes in zwei aufeinander senkrecht stehenden Schnittebenen.

Eine weitere intrakavitäre Ultraschallsonde ist aus der US-PS 4 543 960 bekannt. Diese Ultraschallsonde ist als Transösophagussonde für kardiologische Untersuchungen ausgebildet. Der Abtastkopf der Sonde umfaßt einen Innenraum, in dem ein Phased-Array drehbar gelagert ist, um in verschiedenen Ebenen abtasten zu können. Das Array wird dabei in der Ebene der Wandlerelemente, die das Array bilden, gedreht. Im Nahbereich ist jedoch die Auflösung des Phased-Arrays gering, so daß anatomische Details in unmittelbarer Nähe des Abtastkopfes nur unzureichend abgebildet werden.

Der Erfindung liegt nun die Aufgabe zugrunde, eine intrakavitäre Ultraschallsonde mit einem großen Abtastfeld und einer guten Auflösung im Nahbereich anzugeben, mit der verschiedene Schnittbilder des Untersuchungsgebietes erstellt werden können, die einen gemeinsamen Ursprung besitzen.

Die Aufgabe wird dadurch gelöst, daß das Endteil um eine Drehachse drehbar in dem Einführabschnitt gelagert ist und daß die Drehachse radial in bezug zum Wandlerarray ausgerichtet ist. Durch die radiale Ausrichtung der Drehachse in bezug zum Wandlerarray können von dem Untersuchungsgebiet mehrere Schnittbilder mit einem gemeinsamen Ursprung erstellt werden. Damit kann die Anatomie des Untersuchungsgebiets besser erschlossen werden. Insbesondere können auch zwei aufeinander senkrecht stehende Schnittbilder erstellt werden, die einen gemeinsamen Ursprung aufweisen und die diagnostisch besonders interessant sind.

Eine vorteilhafte Ausgestaltung zeichnet sich dadurch aus, daß die Ultraschallsonde als Prostatasonde ausgebildet ist. Insbesondere bei der Ultraschall-Abtastung der Prostata vom Rektum aus ist durch Verwendung eines Curved-Arrays eine gute Auflösung im Nahbereich gegeben.

Eine weitere vorteilhafte Ausgestaltung zeichnet sich dadurch aus, daß zwischen dem Einführabschnitt und dem Endteil eine flüssigkeitsdichte Dichtung mit einem geringen Reibwiderstand angeordnet ist. Obwohl bei einer Untersuchung die Ultraschallsonde mit einer Schutzhülle versehen wird, ist durch die Dichtung vermieden, daß bei einer Beschädigung der Schutzhülle Körperflüssigkeiten in die Ultraschallsonde eindringen. Außerdem verhindert die Dichtung das Eindringen von flüssigen Reinigungs- und Desinfektionsmitteln, die toxisch sein können.

Eine weitere vorteilhafte Ausgestaltung zeichnet sich dadurch aus, daß der Einführabschnitt eine Längsachse aufweist und daß die Drehachse die Längsachse schneidet. Durch die seitliche Ausrichtung der Abtastebenen ist die Anatomie der Prostata einer Ultraschallabtastung vom Rektum aus besonders leicht zugänglich.

Eine weitere vorteilhafte Ausgestaltung zeichnet sich dadurch aus, daß das Endteil eine kalottenförmige Oberfläche aufweist, in der das Wandlerarray angeordnet ist, und daß die Drehachse die Symmetrieachse des Endteils darstellt. Durch die kalottenförmige Oberfläche ist zum einen gewährleistet, daß die Ultraschallsonde ohne Schwierigkeit in eine Körperhöhle eingeführt werden kann. Zum andern erleichtert die kalottenförmige Oberfläche Drehungen des konvexen Wandlerarrays in der Untersuchungsposition. Dadurch, daß das Wandlerarray in der kalottenförmigen Oberfläche angeordnet ist, ergibt sich der Vorteil, daß der Krümmungsradius und damit die Auflösung im Nahbereich so groß wie möglich wird.

Ein besonders großes Abtastfeld ist bei einer weiteren vorteilhaften Ausgestaltung dadurch verwirklicht, daß die Oberfläche eine Halbkugeloberfläche ist, daß das Wandlerarray auf einem Längenkreisbogen angeordnet ist und daß der Längenkreisbogen annähernd die Länge eines Halbkreises aufweist.

Weitere vorteilhafte Ausgestaltungen ergeben sich aus den Unteransprüche und der Beschreibung von drei Ausführungsbeispielen anhand von drei Figuren. Es zeigen:
- FIG 1: in einem Teillängsschnitt eine als Prostatasonde ausgebildete intrakavitäre Ultraschallsonde, bei der die Drehachse eines konvexen Wandlerarrays unter 45° zur Längsachse der Sonde ausgerichtet ist und bei der das Verstellmittel zum Verdrehen des Wandlerarrays als Gestänge mit Kardangelenken ausgeführt ist,
- FIG 2: eine Prostatasonde ähnlich wie in FIG 1, jedoch mit einem als Seilantrieb ausgeführten Verstellmittel und
- FIG 3: in einer vergrößerten Schnittdarstellung den vorderen Endabschnitt einer Prostatasonde, bei der die Drehachse um 90° zur Längsachse der Sonde ausgerichtet ist.

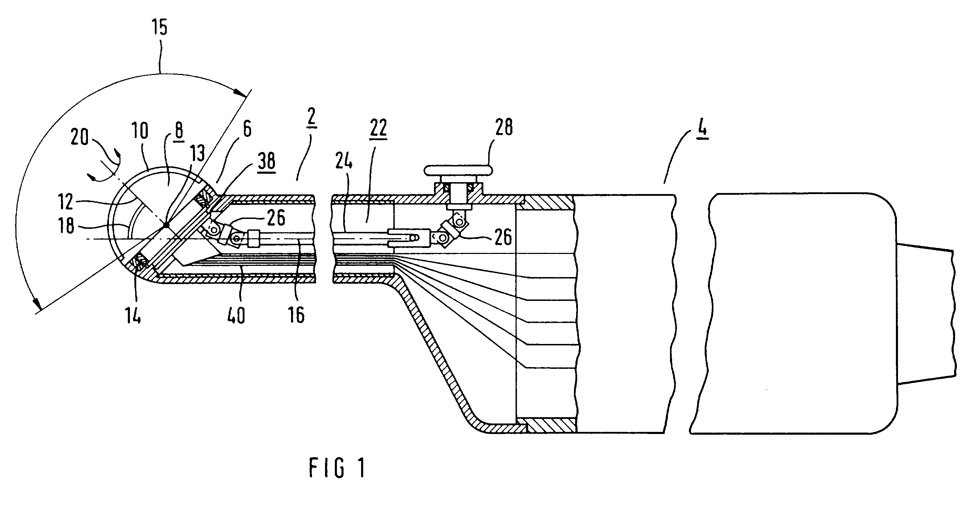

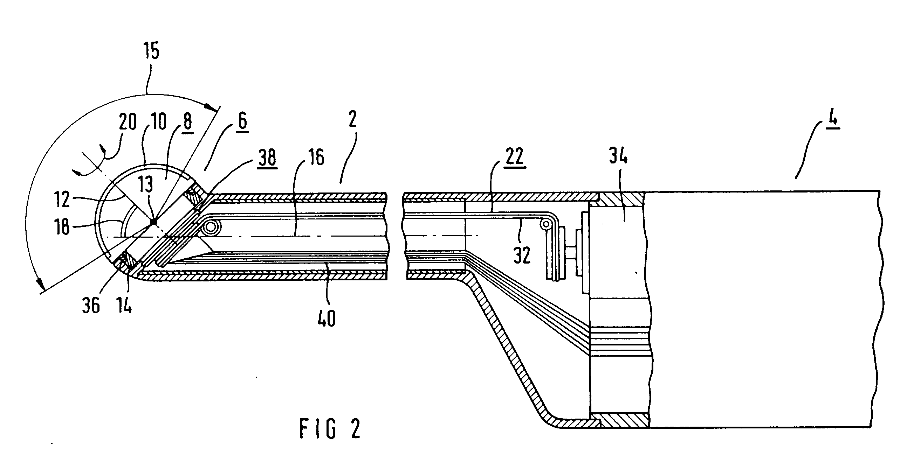

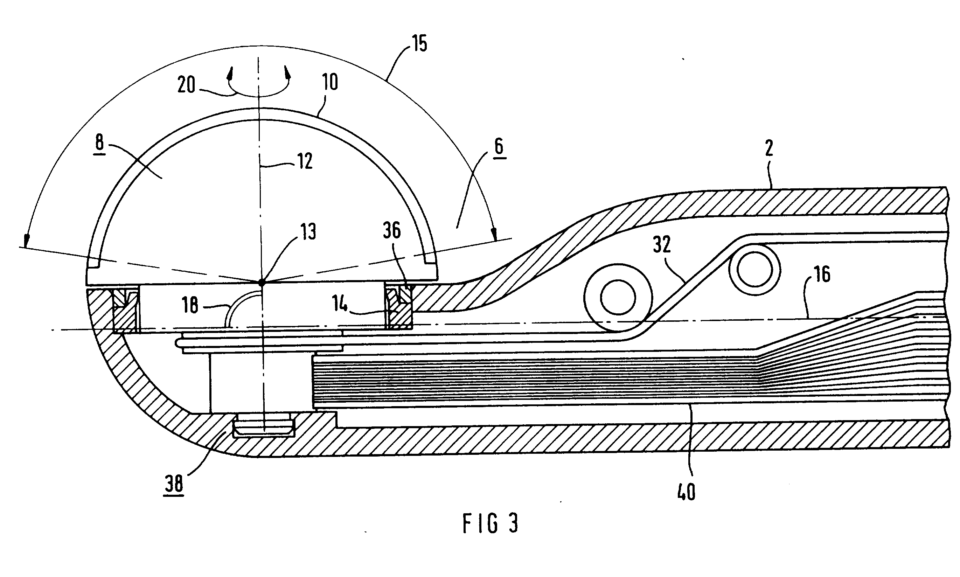

Sowohl die in FIG 1 als auch die in FIG 2 gezeigte Ultraschall-Prostatasonde umfaßt einen geraden Einführabschnitt 2, der an seinem proximalen Ende in einen Handgriff 4 übergeht. Der Einführabschnitt 2 ist ungefähr 150 mm lang und weist einen kreisförmigen Querschnitt mit einem Durchmesser von 15 mm auf. Am distalen Ende 6 des Einführabschnitts 2 ist ein Endteil 8 angeordnet, das ein konvexes Wandlerarray 10 umfaßt. Das Endteil 8 ist um eine Drehachse 12 drehbar in dem Einführabschnitt 2 gelagert. Die Lagerung ist anhand von FIG 3 genauer beschrieben. Die Drehachse 12 ist radial und symmetrisch in bezug zum Wandlerarray 10 ausgerichtet. Alle zueinander verdrehten Abtastebenen des Wandlerarrays 10 besitzen einen gemeinsamen Ursprung 13, der gleichzeitig der Punkt ist, von dem die Abtaststrahlen in den Abtastebenen scheinbar ausgehen. Zwischen dem Endteil 8 und dem distalen Ende 6 des Einführabschnitts 2 ist eine flüssigkeitsdichte Dichtung 14 mit einem geringen Reibwiderstand angeordnet. Hier ist die Dichtung 14 als 0-Ring ausgebildet, während in FIG 2 die Dichtung 14 eine Lippendichtung ist.

Das Abtastfeld 15 des Wandlerarrays 10 ist unter einem Winkel 18 von 45° schräg nach vorne ausgerichtet. Daher schneidet die Drehachse 12 eine Längsachse 16 des Einführabschnitts 2 unter diesem Winkel 18.

Das Endteil 8 ist als Halbkugel mit einem Durchmesser von 20 mm ausgebildet, in deren Oberfläche das Wandlerarray 10 angeordnet ist. Das Wandlerarray 10 ist auf einem Längenkreisbogen auf der Halbkugeloberfläche angeordnet, wobei der Längenkreisbogen annähernd die Länge eines Halbkreises besitzt. Die Drehachse 12 fällt somit mit der Symmetrieachse des Endteils 8 zusammen. Insgesamt besteht das Wandlerarray 10 hier aus 192 Elementarwandlern, die zur Sektorabtastung gruppenweise fortgeschaltet werden.

Der gesamte Drehwinkel 20, um den das Wandlerarray drehbar ist, beträgt 180°. Damit kann das vollständige Volumen der Prostata in Schnittbildern dargestellt werden. Daher können auch gegebenenfalls 3D-Datensätze erstellt werden, aus denen beliebig liegende Schnittbilder elektronisch gebildet werden können.

Zum Verdrehen des Endteils 8 und damit zur Auswahl der Abtastebene sind im Innenraum des Einführabschnitts 2 Verstellmittel 22 vorgesehen, die in FIG 1 ein Gestänge 24 mit Kardangelenken 26 und ein Handrad 28 umfassen.

Die in FIG 2 dargestellte Prostatasonde unterscheidet sich von in FIG 1 dargestellten durch die Ausführung der Verstellmittel 22. Hier ist zum Verdrehen und Einstellen der Abtastebenen ein Seilantrieb 32 mit Umlenkrollen vorgesehen, der von einem elektrischen Schrittmotor 34 angetrieben wird.

Bei der in FIG 3 dargestellten Ausführungsform der Prostatasonde ist die Drehachse 12 senkrecht zur Längsachse 16 ausgerichtet. Dazu ist das distale Ende 6 des ansonsten kreisrunden Einführabschnitts 2 parallel zur Längsachse 16 abgeflacht. Hier wird ebenso wie in FIG 2 ein Seilantrieb 32 mit Umlenkrollen zur Verdrehung des Endteils 8 benutzt.

Die Dichtung 14 ist hier als Lippendichtung ausgeführt, die mit einem Haltering 36 in einer kreisförmigen Ausfräsung am abgeflachten distalen Ende 6 gehalten wird.

Die Lagerung des Endteils 8 erfolgt über ein Schnapplager 38, das aus einem am Endteil 8 ausgebildeten Zapfen und einer entsprechend ausgebildeten Ausnehmung im distalen Endes 6 besteht. Ähnlich ist auch die Lagerung des Endteils in den FIG 1 und 2 ausgeführt.

In allen Ausführungsformen ist die elektrische Verbindung der einzelnen Wandlerelemente im Wandlerarray 10 zu einer Signalverarbeitung (hier nicht dargestellt) über eine flexible Leiterplatte 40 hergestellt, die sich durch den Innenraum des Einführabschnitts 2 bis zum Handgriff 4 erstreckt.

Es soll noch erwähnt werden, daß der Einführabschnitt 2 und das Endteil 8 aus einem desinfizierbaren Kunststoff bestehen, wobei sich für den Einführabschnitt 2 PTFE als günstig herausgestellt hat. Außerdem lassen sich bei der Verwendung dieses Kunststoffs besonders reibungsarme Lagerungen aufbauen, wenn die anderen Bauteile, wie z.B. der Zapfen im Schnapplager 38, aus Polyamid sind.

## Patentansprüche

1. Intrakavitäre Ultraschallsonde zur Erzeugung von Schnittbildern eines Untersuchungsgebietes mit einem Einführabschnitt (2), der distal in einem Endteil (8) endet, welches Endteil (8) ein konvexes Wandlerarray (10) umfaßt, **dadurch gekennzeichnet,** daß das Endteil (8) um eine Drehachse (12) drehbar in dem Einführabschnitt (2) gelagert ist und daß die Drehachse (12) radial in bezug zum Wandlerarray (10) ausgerichtet ist.

2. Ultraschallsonde nach Anspruch 1, **dadurch gekennzeichnet,** daß die Ultraschallsonde als Prostatasonde ausgebildet ist.

3. Ultraschallsonde nach Anspruch 1 oder 2, **dadurch gekennzeichnet,** daß die Drehachse (12) symmetrisch in bezug zum Wandlerarray (10) ausgerichtet ist.

4. Ultraschallsonde nach einem der Ansprüche 1 bis 3, **dadurch gekennzeichnet,** daß zwischen dem Einführabschnitt (2) und dem Endteil (8) eine flüssigkeitsdichte Dichtung (14) mit einem geringen Reibwiderstand angeordnet ist.

5. Ultraschallsonde nach einem der Ansprüche 1 bis 4, **dadurch gekennzeichnet,** daß der Einführabschnitt (2) eine Längsachse (16) aufweist und daß die Drehachse (12) die Längsachse (16) schneidet.

6. Ultraschallsonde nach Anspruch 5, **dadurch gekennzeichnet,** daß die Drehachse (12) die Längsachse (16) schräg schneidet.

7. Ultraschallsonde nach Anspruch 5, **dadurch gekennzeichnet,** daß die Drehachse (12) die Längsachse (16) unter einem rechten Winkel schneidet.

8. Ultraschallsonde nach einem der Ansprüche 1 bis 7, **dadurch gekennzeichnet,** daß das Enteil (8) eine kalottenförmige Oberfläche aufweist, in der das Wandlerarray (10) angeordnet ist, und daß die Drehachse (12) die Symmetrieachse des Endteils (8) darstellt.

9. Ultraschallsonde nach Anspruch 8, **dadurch gekennzeichnet,** daß die Oberfläche eine Halbkugeloberfläche ist, daß das Wandlerarray (10) auf einem Längenkreisbogen des Endteils (8) angeordnet ist und daß der Längenkreisbogen annähernd die Länge eines Halbkreises aufweist.

10. Ultraschallsonde nach einem der Ansprüche 1 bis 9, **dadurch gekennzeichnet,** daß Verstellmittel (22) mit dem Endteil (8) verbunden sind, über die das Endteil (8) um seine Drehachse (12) drehbar ist.

11. Ultraschallsonde nach Anspruch 10, **dadurch gekennzeichnet,** daß die Verstellmittel (22) ein Gestänge (24) mit Kardangelenken (26) umfassen.

12. Ultraschallsonde nach Anspruch 10, **dadurch gekennzeichnet,** daß die Verstellmittel (22) einem Seilantrieb (32) umfassen.

13. Ultraschallsonde nach einem der Ansprüche 10 bis 12, **dadurch gekennzeichnet,** daß die Verstellmittel (22) ein Handrad (24) umfassen.

14. Ultraschall-Prostatasonde nach einem der Ansprüche 10 bis 12, **dadurch gekennzeichnet,** daß die Verstellmittel (22) einen elektrischen Stellmotor (34) umfassen.

## Claims

1. An intracavitary ultrasound probe for producing tomograms of an examination region, having an introduction section (2), the introduction section terminating distally in an endpiece (8), the endpiece (8) containing a convex transducer array (10), characterized in that the endpiece (8) is rotatably mounted in the introduction section (2) relative to a swivelling axis (12), and the swivelling axis (12) is aligned radially relative to the transducer array (10).

2. An ultrasound probe as claimed in claim 1, characterized in that the ultrasound probe is fashioned as a prostate probe.

3. An ultrasound probe as claimed in claims 1 and 2, characterized in that the swivelling axis (12) is aligned symmetrically relative to the transducer array (10).

4. An ultrasound probe as claimed in any one of claims 1 to 3, characterized in that a liquid-tight seal (14), having a low frictional resistance, is disposed between the introduction section (2) and the endpiece (8).

5. An ultrasound probe as claimed in any one of claims 1 to 4, characterized in that the introduction section (2) has a longitudinal axis (16), and the swivelling axis (12) intersects the longitudinal axis (16).

6. An ultrasound probe as claimed in claim 5, characterized in that the swivelling axis (12) obliquely intersects the longitudinal axis (16).

7. An ultrasound probe as claimed in claim 5, characterized in that the swivelling axis (12) intersects the longitudinal axis (16) at a right angle.

8. An ultrasound probe as claimed in any one of claims 1 to 7, characterized in that the endpiece (8) has a dome-shaped surface within which the transducer array (10) is disposed, and the swivelling axis (12) forms the axis of symmetry of the endpiece (8).

9. An ultrasound probe as claimed in claim 8, characterized in that the surface is a hemispherical surface, the transducer array (10) is disposed on a longitudinal arc of a circle of the endpiece (8), and the longitudinal arc of a circle approximates the length of a semicircle.

10. An ultrasound probe as claimed in any one of claims 1 to 9, characterized in that adjustment means (22), with which the endpiece (8) is rotatable around the swivelling axis (12) thereof, are connected with the endpiece (8).

11. An ultrasound probe as claimed in claim 10, characterized in that the adjustment means (22) comprise a rod (24) having Cardan joints (26).

12. An ultrasound probe as claimed in claim 10, characterized in that the adjustment means (22) comprise a cable drive (32).

13. An ultrasound probe as claimed in any one of claims 10 to 12, characterized in that the adjustment means (22) comprise a handwheel (28).

14. An ultrasound probe as claimed in any one of claims 10 to 12, characterized in that the adjustment means (22) comprise an electrical stepping motor (34).

## Revendications

1. Sonde intracavitaire à ultrasons destinée à produire des tomographies d'une zone à analyser, comportant une partie (2) d'introduction qui se termine distalement en un élément (8) d'extrémité ayant un réseau (10) convexe de transducteurs, **caractérisée** en ce que l'élément (8) d'extrémité est monté à rotation autour d'un axe (12) de rotation dans la partie (2) d'introduction, et en ce que l'axe (12) de rotation est orienté radialement par rapport au réseau (10) de transducteurs.

2. Sonde à ultrasons suivant la revendication 1, **caractérisée** en ce que la sonde à ultrasons sous la forme d'une sonde de prostate.

3. Sonde à ultrasons suivant la revendication 1 ou 2, **caractérisée** en ce que l'axe (12) de rotation est orienté symétriquement par rapport au réseau (10) de transducteurs.

4. Sonde à ultrasons suivant l'une des revendications 1 à 3, **caractérisée** en ce qu'un joint (14) d'étanchéité à l'épreuve des liquides, à faible résistance de frottement, est disposé entre la partie (2) d'introduction et l'élément (8) d'extrémité.

5. Sonde à ultrasons suivant l'une des revendications 1 à 4, **caractérisée** en ce que la partie (2) d'introduction possède un axe (16) longitudinal, et en ce que l'axe (12) de rotation coupe l'axe (16) longitudinal.

6. Sonde à ultrasons suivant la revendication 5, **caractérisée** en ce que l'axe (12) de rotation coupe en oblique l'axe (16) longitudinal.

7. Sonde à ultrasons suivant la revendication 5, **caractérisée** en ce que l'axe (12) de rotation coupe à angle droit l'axe (16) longitudinal.

8. Sonde à ultrasons suivant l'une des revendications 1 à 7, **caractérisée** en ce que l'élément (8) d'extrémité possède une surface en forme de calotte, dans laquelle est disposé le réseau (10) de transducteurs, et en ce que l'axe (12) de rotation constitue l'axe de symétrie de l'élément (8) d'extrémité.

9. Sonde à ultrasons suivant la revendication 8, **caractérisée** en ce que la surface est une surface hémisphérique, en ce que le réseau (10) de transducteurs est disposé sur un arc de cercle longitudinal de l'élément (8) d'extrémité, et en ce que l'arc de cercle longitudinal a environ la longueur d'un demi-cercle.

10. Sonde à ultrasons suivant l'une des revendications 1 à 9, **caractérisée** en ce que des moyens (22) de réglage sont reliés à l'élément (8) d'extrémité, qui permettent de faire tourner l'élément (8) d'extrémité autour de son axe (12) de rotation.

11. Sonde à ultrasons suivant la revendication 10, **caractérisée** en ce que les moyens (22) de réglage comprennent une tringlerie (24) pourvue d'articulations (26) à la Cardan.

12. Sonde à ultrasons suivant la revendication 10, **caractérisée** en ce que les moyens (22) de réglage comprennent une commande (32) par câble.

13. Sonde à ultrasons suivant l'une des revendications 10 à 12, **caractérisée** en ce que les moyens (22) de réglage comprennent une roue (28) à main.

14. Sonde de prostate à ultrasons suivant l'une des revendications 10 à 12, **caractérisée** en ce que les moyens (22) de réglage comprennent un servomoteur (34) électrique.
